# FASCICULE DE BREVET EUROPEEN

(11) **EP 2 816 968 B1**
(45) Date de publication et mention de la délivrance du brevet: **22.08.2018**
(21) Numéro de dépôt: 13704986.2
(22) Date de dépôt: 21.02.2013
(51) Int. Cl.: A61F 2/00

(54) **SYSTEME DE CONTROLE D'UN DISPOSITIF IMPLANTABLE A PARTIR D'ORDRES EXECUTES PAR UN UTILISATEUR**
SYSTEM ZUR STEUERUNG EINER IMPLANTIERBAREN VORRICHTUNG BASIEREND AUF VON EINEM BENUTZER AUSGEGEBENEN BEFEHLEN
SYSTEM FOR CONTROLLING AN IMPLANTABLE DEVICE ON THE BASIS OF COMMANDS ISSUED BY A USER

(30) Priorité: 21.02.2012 US 201261601311 P; 06.04.2012 FR 1253204
(43) Date de publication de la demande: 31.12.2014
(73) Titulaire: Uromems, 38000 Grenoble (FR)
(72) Inventeur: LAMRAOUI, Hamid, 38000 Grenoble (FR)
(74) Mandataire: Regimbeau
(86) Numéro de dépôt international: PCT/EP2013/053469
(87) Numéro de publication internationale: WO 2013/124362

(56) Documents cités:
- EP-A1- 1 676 543
- WO-A1-00/19939
- US-A1- 2003 212 306
- US-A1- 2004 172 087
- US-B1- 6 471 635

## Description

### DOMAINE DE L'INVENTION

La présente invention concerne un système permettant le contrôle externe d'un dispositif implantable sans périphérique externe. Cette invention offre un contrôle ergonomique et sûr d'un implant médical par le patient et le personnel médical.

### ETAT DE LA TECHNIQUE

La communication sans fil, utilisée de deux façons différentes (induction électromagnétique et radiofréquence), est une méthode courante pour le contrôle et la configuration de dispositifs médicaux implantables fonctionnant sur pile ou batterie. La configuration du dispositif nécessite une base sans fil externe connectée à une unité de contrôle intégrant une interface utilisateur. Cette procédure est généralement effectuée par le médecin afin d'appliquer un traitement adapté au patient. Pour certains dispositifs implantables actifs, le patient peut contrôler des paramètres précis, délimités par le médecin, afin de modifier partiellement la thérapie. Par exemple, les patients porteurs d'un dispositif médical implantable actif pour le traitement de douleur peuvent atténuer plus ou moins la douleur grâce à une télécommande. De la même manière, le neuromodulateur implantable du nerf sacré S3, pour la stimulation électrique vésical, peut être contrôlé par le patient à l'aide d'une télécommande externe afin d'activer ou de désactiver le dispositif implantable ou de modifier la neurostimulation du nerf sacré. Une télécommande peut également être utilisée par le patient pour diverses applications médicales implantables, par exemple, pour le contrôle des sphincters artificiels, des dispositifs de délivrance de médicaments, des neurostimulateurs ou encore des anneaux gastriques actifs.

Un inconvénient de cette méthode est que le patient doit porter en permanence sur lui la télécommande externe. Étant donné que l'alimentation est nécessaire dans la télécommande, l'autonomie doit être suffisamment longue pour éviter à l'utilisateur d'être incapable d'effectuer des contrôles à distance du dispositif implanté. Un autre inconvénient de cette méthode est qu'en cas d'urgence, sans une télécommande adaptée, il est impossible de contrôler l'implant actif.

L'utilisation d'un aimant extracorporel est un autre moyen de contrôle d'un dispositif implantable actif. En plaçant l'aimant au-dessus de la zone où se trouve le dispositif implantable, cela peut activer ou désactiver le dispositif implantable ou certaines de ses fonctions, ou modifier certains paramètres de l'appareil.

Encore une fois, sans l'aimant de contrôle (et sans une télécommande appropriée) l'utilisateur est incapable de contrôler le dispositif implantable. En outre, lorsque le patient se trouve dans un environnement comportant des perturbations électriques, électromagnétiques ou magnétiques, des activations/désactivations aléatoires du dispositif implantable ou certaines de ses fonctions peuvent se produire. Si le patient vit ou travaille dans ce type d'environnement, le contrôle par aimant doit être désactivé par le clinicien, privant le patient de cette fonction.

Les méthodes présentées ci-dessus représentent un réel problème d'ergonomie pour le patient qui doit porter en permanence un objet afin de contrôler le dispositif implanté. En outre, dans le cas d'une télécommande sans fil, le patient doit s'assurer que le niveau d'énergie de la télécommande est suffisamment élevé pour pouvoir l'utiliser. En conséquence, et en plus d'un problème d'ergonomie, cet inconvénient peut entraîner un stress supplémentaire pour le patient.

L'absence de sécurité est un autre inconvénient majeur de ces méthodes. En effet, sans un dispositif approprié, le dispositif implantable ne peut être contrôlé en cas d'urgence. L'objet de la présente invention est de fournir une solution sûre, fiable, simple et ergonomique pour commander un dispositif implantable dans le but de résoudre les inconvénients mentionnés ci-dessus.

### EXPOSÉ DE L'INVENTION

En vue de ce qui précède, un but de la présente invention est de fournir un système pour commander, sans un objet externe, un dispositif déjà implanté dans une personne ou un organisme animal par l'envoi de commandes au dispositif et comprenant les étapes suivantes :
- appliquer à une partie du corps comprenant au moins un capteur déjà implanté un motif d'au moins une action mécanique selon un code prédéterminé, l'action mécanique étant mesurée par au moins un capteur et convertie en au moins un signal,
   où le code prédéterminé comprend au moins une action mécanique, définie par ses caractéristiques d'au moins un signal correspondant et au moins une information supplémentaire détectable par au moins un capteur,
- identifier chaque action mécanique dans une portion d'au moins un signal mesuré et traité,
- comparer les caractéristiques de chaque portion d'au moins un signal avec un modèle de référence correspondant aux caractéristiques du signal,
- déterminer, à partir de chaque comparaison du signal, si toutes les actions mécaniques mesurées appartiennent au code prédéterminé, et
- si le motif appliqué correspond à un code prédéterminé, envoyer une commande prédéterminée correspondante au dispositif implanté.

Le modèle de référence des caractéristiques du signal peut être défini par l'amplitude du signal, des données statistiques, la forme d'onde, les caractéristiques en fréquence, la durée ou tous paramètres qui peuvent caractériser un signal spécifique. Le modèle de référence des caractéristiques du signal peut correspondre à une ou plusieurs caractéristiques d'un signal qui peuvent être utilisées pour identifier un signal spécifique. Dans un mode de réalisation préféré, l'information supplémentaire détectable comprend l'absence, pendant une durée prédéterminée, de toute action mécanique ayant une amplitude au-dessus d'un seuil prédéterminé.

Dans un autre mode de réalisation préféré, l'information supplémentaire détectable comprend une position prédéterminée pendant une durée prédéterminée. Avantageusement, chaque information supplémentaire détectable et chaque action mécanique ont une durée prédéterminée avec des tolérances de durées inférieures et supérieures prédéterminées.

Les informations supplémentaires peuvent être définies, en fonction du capteur utilisé, comme étant le signal de sortie, ou un paramètre du signal de sortie, d'au moins un capteur inférieur à un seuil prédéterminé (ci-après dénommé « silence ») et/ou correspondant à une position spécifique du patient, par exemple le patient est immobile ou est couché. Les actions mécaniques sont détectées lorsque le ou les signaux de sortie du ou des capteurs franchissent un seuil prédéterminé, et quand le ou les signaux correspondant à l'action mécanique ont les mêmes caractéristiques que le modèle de référence enregistré des caractéristiques du signal correspondant qui est stocké dans une mémoire. Une erreur acceptable sur la comparaison entre les caractéristiques du signal est prise en compte.

Dans une configuration privilégiée, la méthode comprend en outre les étapes consistant à :
a) détecter au moins une information supplémentaire dans une partie d'au moins un signal qui a un niveau inférieur à un seuil prédéterminé,
b) détecter au moins une action mécanique dans une portion d'au moins un signal qui a un niveau au-dessus d'un seuil prédéterminé durant une période comprenant un minimum et un maximum de temps, correspondant respectivement à une durée d'action mécanique minimale et maximale après un silence prédéterminé,
c) stocker chaque signal dans une mémoire,
d) répéter les étapes (a) à (c) pour chaque partie de chaque signal jusqu'à la fin du motif à détecter,
e) si le motif ne correspond pas à un code prédéterminé, reprendre à l'étape (a) à tout moment jusqu'à la détection d'un motif correspondant à un code prédéterminé.

Lorsqu'un motif correspond à un code prédéterminé, chaque signal stocké en mémoire est comparé à son modèle de référence des caractéristiques du signal correspondant. Si tous les signaux correspondent à des actions mécaniques attendues mesurées par au moins un capteur, le code prédéterminé est validé et un ordre correspondant est envoyé au dispositif implanté.

Le modèle de référence des caractéristiques du signal correspondant de chaque action mécanique peut être mis à jour périodiquement.

C'est encore un autre objet de cette invention de paramétrer des codes spécifiques et prédéterminés d'actions mécaniques exercées par une personne et géré par une unité de contrôle implantable, afin de faire exécuter au dispositif implantable des ordres prédéterminés correspondant au code exécuté.

C'est encore un objet de la présente invention de mesurer les actions mécaniques en utilisant au moins un capteur implanté au préalable dans le corps du patient.

Dans une réalisation privilégiée, l'ordre prédéterminé est sélectionné dans le groupe comprenant l'activation ou la désactivation du dispositif implanté, l'activation ou la désactivation d'au moins une fonction du dispositif implanté, la modification d'au moins un paramètre du dispositif implanté, la modification de la thérapie appliquée par le dispositif implanté, la modification de la forme du dispositif implanté et l'activation ou la désactivation d'au moins une fonctionnalité de sécurité du dispositif implanté.

Un retour à l'utilisateur peut-être être généré lorsqu'un ordre prédéterminé est envoyé au dispositif implanté.

Avantageusement, l'action mécanique est sélectionnée dans le groupe comprenant des percussions manuelles externe au niveau du site d'implantation du ou des capteurs, des contractions musculaires, des vibrations, des augmentations de la pression d'une cavité intracorporelle et une augmentation de la pression sur la peau au-dessus du site d'implantation du ou des capteurs.

Le dispositif implanté peut être un sphincter artificiel et l'ordre prédéterminé peut comporter l'ouverture ou la fermeture d'un élément occlusif du sphincter artificiel.

Dans une réalisation privilégiée, le dispositif implanté comprend :
- au moins un capteur implantable dans un corps animal ou humain et adapté pour mesurer des actions mécaniques,
- une unité de contrôle implantable dans un corps animal ou humain et adaptée pour le traitement d'au moins un signal provenant d'au moins un capteur ; la détection d'un code prédéterminé ; l'identification des actions mécaniques dans une portion d'au moins un signal mesuré ; la comparaison des caractéristiques de chaque portion d'au moins un signal avec un modèle de référence des caractéristiques du signal correspondant ; la détermination, par la comparaison du ou des signaux, si l'action mécanique mesurée appartient bien au code prédéterminé et l'envoi d'un ordre prédéterminé au dispositif implanté si le code est validé.

Dans une réalisation privilégiée, le capteur implanté dans le corps du patient est adapté pour la mesure ou la détection de pressions, chocs, accélérations, vibrations ou contractions musculaires. Le capteur peut être sélectionné dans le groupe comprenant un accéléromètre 1, 2 ou 3 axes, un gyroscope, un système de localisation spatiale, un capteur de pression et un commutateur électrique.

Dans une autre mode de réalisation, au moins des capteurs n'est pas alimenté électriquement et génère un signal électrique lorsqu'une action mécanique est appliquée sur le ou les capteurs. Le capteur peut être basé sur un fonctionnement piézoélectrique ou magnétique.

Le dispositif implantable peut être équipé d'un système de communication sans fil entre le dispositif implantable et une base sans fil externe afin de configurer les paramètres de la détection des codes prédéterminés.

Il est à noter que la méthode n'inclut pas l'étape d'implantation du dispositif implantable dans le corps du patient. Elle ne comprend pas non plus l'étape d'implantation du ou des capteurs et de l'unité de contrôle si ces derniers sont distincts du dispositif implantable. Les codes spécifiques sont constitués d'au moins une action mécanique exécutée par une personne, combinée(s) avec au moins une information supplémentaire détectable par au moins un capteur et prédéterminé afin d'être détectés seulement lorsque l'utilisateur en exécute un et ce, par conséquent, pour éviter toute détection intempestive du code.

A la place d'utiliser une télécommande, les patients et les médecins peuvent bénéficier de cette méthode et de l'appareil présenté dans cette invention pour envoyer eux-mêmes directement divers types de commande à un dispositif implantable.

Dans le cas d'un sphincter artificiel, le patient peut contrôler l'ouverture et l'occlusion du sphincter artificiel avec un ou deux codes distinctifs. Il serait également possible d'ajuster le degré de l'occlusion avec un code spécifique.

Dans le cas d'un neurostimulateur, la thérapie peut être modifiée par l'exécution de codes différents selon le traitement souhaité.

Plus généralement, des fonctions d'un dispositif implantable peuvent être, avec des codes prédéterminés, modifiées, désactivées ou activées.

La forme d'un dispositif implantable, tels que celui d'un anneau gastrique, un sphincter artificiel, un implant orthopédique peut être également modifiée lorsqu'une personne exécute un code prédéterminé d'actions mécaniques.

Un autre code peut être mis en oeuvre afin de désactiver le dispositif implantable lorsque cela est souhaité.

En cas d'urgence, lorsqu'aucune télécommande externe adaptée n'est disponible pour contrôler le dispositif implantable, un code d'urgence, connu dans chaque centre clinique, pourrait être exécuté par le personnel médical afin de désactiver les fonctions du dispositif implantable.

Dans une réalisation privilégiée, une routine d'étalonnage automatique est effectuée périodiquement afin d'actualiser les modèles de référence des caractéristiques du signal des actions mécaniques. Lorsqu'un code est exécuté par une personne le ou les signaux de sortie du ou des capteurs sont enregistrés dans la mémoire. Les caractéristiques sont ensuite corrigées et enregistrées dans la mémoire pour constituer un nouveau modèle. Les seuils prédéterminés sont également mis à jour périodiquement de la même manière que les modèles de référence des caractéristiques du signal des actions mécaniques. Les paramètres de détection peuvent être ajustés par un opérateur grâce à une communication sans fil entre le dispositif implantable et une base externe.

Un dispositif externe peut aussi être utilisé pour reproduire le motif des codes d'actions mécaniques afin par exemple de configurer la sensibilité du système de détection. Un procédé de détection d'une commande d'activation d'un dispositif implantable par un utilisateur, susceptible d'être mis en oeuvre dans le système décrit plus comprend les étapes suivantes :
- réception à partir d'un ou plusieurs capteurs du dispositif, d'un signal de commande composé d'une séquence de valeurs, certaines valeurs étant représentatives d'actions mécaniques exercées volontairement par l'utilisateur sur le capteur et d'autres valeurs étant représentatives d'une information supplémentaire, telle qu'une durée entre deux actions mécaniques successives exercées sur le ou les capteurs,
- réception, à partir d'une mémoire du dispositif, d'un ou plusieurs signaux de référence,
- estimation d'un indice de similarité par comparaison d'au moins une portion du signal de commande avec au moins une portion du signal de référence,
- si l'indice de similarité est supérieur à une valeur seuil, reconnaissance d'un code prédéterminé d'activation du dispositif.

Si ledit code prédéterminé est reconnu, un ordre d'actionnement du dispositif peut alors être émis.

### DESCRIPTION DES FIGURES

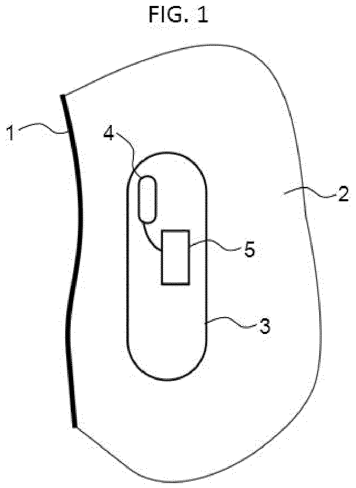

La FIG. 1 illustre une configuration du système de détection des actions mécaniques exécutées par une personne. Dans cette configuration, le capteur et l'unité de contrôle sont intégrés à l'intérieur du boîtier du dispositif implantable. L'unité de contrôle peut être l'unité de contrôle du dispositif implantable prenant en charge la méthode de détection des actions mécaniques exécutées dans un code prédéterminé par l'utilisateur.

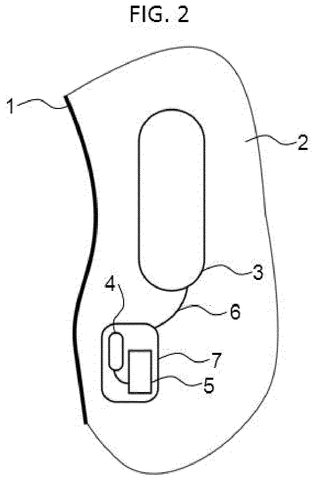

La FIG. 2 illustre une autre configuration du système de détection des actions mécaniques exécutées par une personne. Dans cette configuration, le capteur et l'unité de contrôle sont implantés mais sont intégrés à l'intérieur d'un boîtier dédié et séparé communiquant grâce à une communication soit filaire, soit sans fil avec le dispositif implantable.

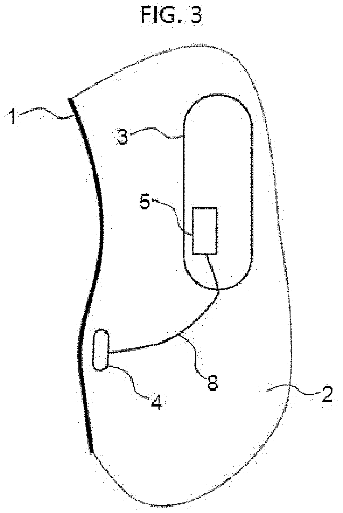

La FIG. 3 illustre une autre configuration du système de détection des actions mécaniques exécutées par une personne. Dans cette configuration, le capteur est intégré à l'intérieur d'un boîtier dédié communiquant grâce à une communication soit filaire, soit sans fil avec le dispositif implantable. L'unité de contrôle peut être l'unité de contrôle du dispositif implantable prenant en charge la méthode de détection des actions mécaniques exécutées dans un code prédéterminé par l'utilisateur.

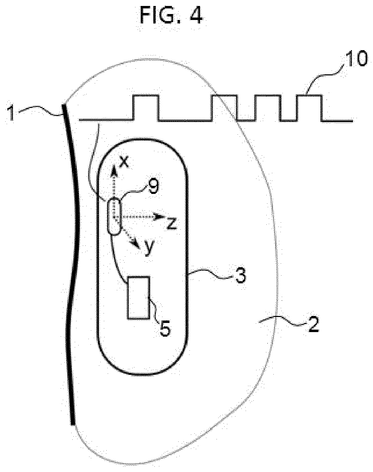

La FIG. 4 montre une configuration du système de détection des actions mécaniques exécutées par une personne, où le capteur est un accéléromètre 3 axes. Dans cette configuration, les actions mécaniques sont des accélérations, ou plus généralement des vibrations (par exemple contractions musculaires, mouvements abdominaux...) initiées volontairement par l'utilisateur.

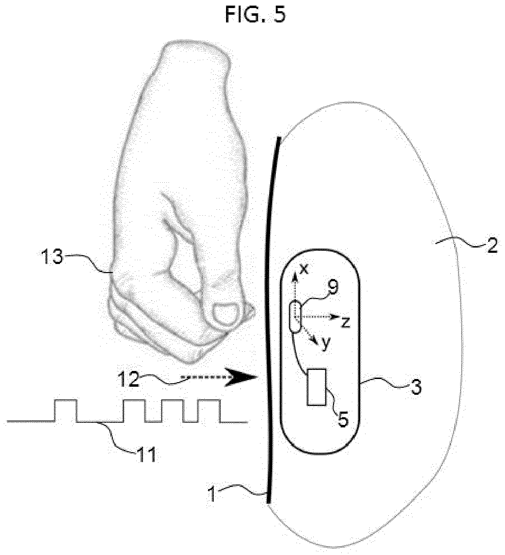

La FIG. 5 est une illustration montrant le contrôle du dispositif implantable par tapotement de la main. Dans cette configuration, l'utilisateur exécute un code en tapotant sur la peau, sur le site d'implantation de l'unité de capteur, afin d'envoyer une commande au dispositif implanté.

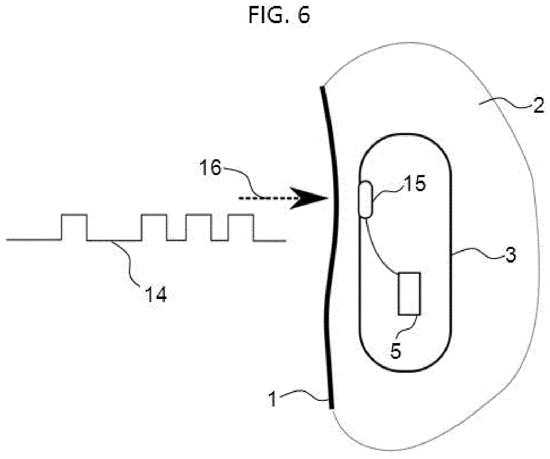

La FIG. 6 est une illustration montrant le contrôle du dispositif implantable en appliquant une pression sur la peau. Dans cette configuration, l'utilisateur exécute un code en appliquant une pression sur la peau, au niveau du site d'implantation du capteur (basé sur un capteur de pression ou un commutateur électrique), afin d'envoyer une commande au dispositif implanté.

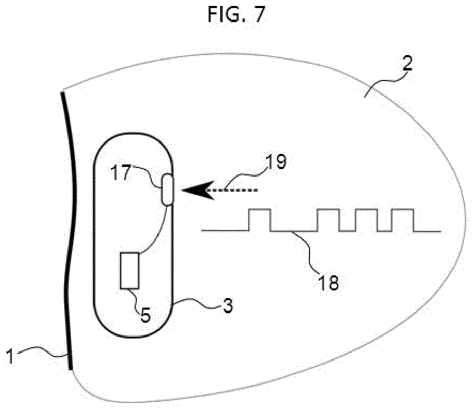

La FIG. 7 est une illustration montrant le contrôle du dispositif implantable en appliquant volontairement une pression à l'intérieur du corps humain. Dans cette configuration, l'utilisateur exécute un code en générant une pression à l'intérieur d'une cavité du corps (par ex. la cavité abdominale), où le capteur se trouve, afin d'envoyer une commande au dispositif implanté.

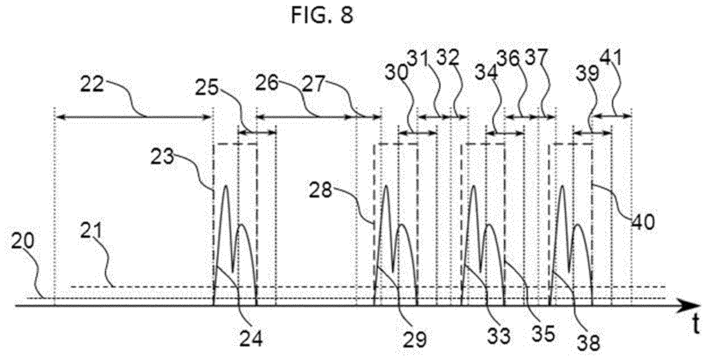

La FIG. 8 illustre les critères de durée qui doivent être respectées afin de valider la première étape de la détection de code. Le code est constitué de plusieurs « silences » du signal correspondant à un niveau de signal inférieur à un seuil prédéterminé et plusieurs actions mécaniques correspondant à un niveau de signal au-dessus d'un seuil prédéterminé.

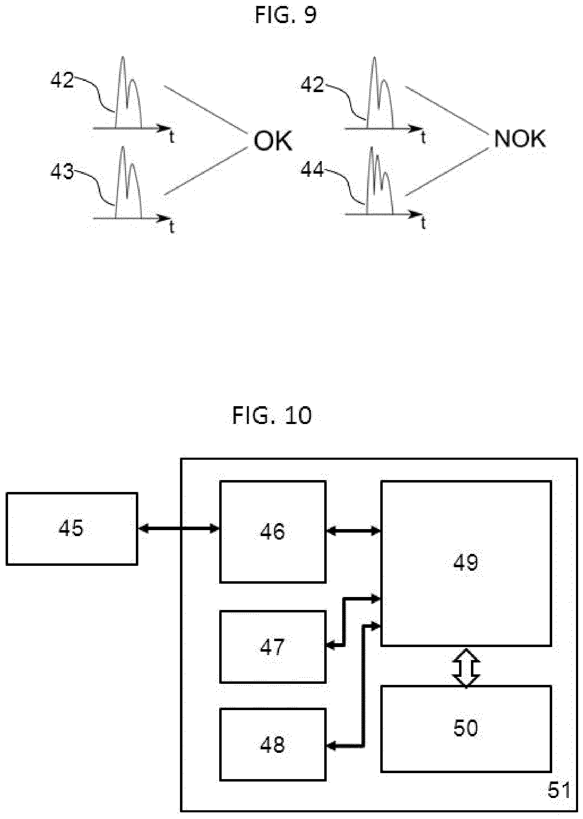

La FIG. 9 illustre la comparaison du signal de l'action mécanique mesuré par le capteur avec un signal de référence dans deux cas. Cela correspond à la deuxième étape de la détection du code. Dans le premier cas (côté gauche de la figure), le signal est similaire, cette étape est donc validée (notation OK). Dans le deuxième cas (à droite de la figure), le signal n'est pas considéré comme similaire au signal de référence, l'étape n'est donc pas validée (notation NOK).

La FIG. 10 est un diagramme blocs du circuit de contrôle. Les fonctions utilisées pour la détection du code sont décrites dans la suite du document.

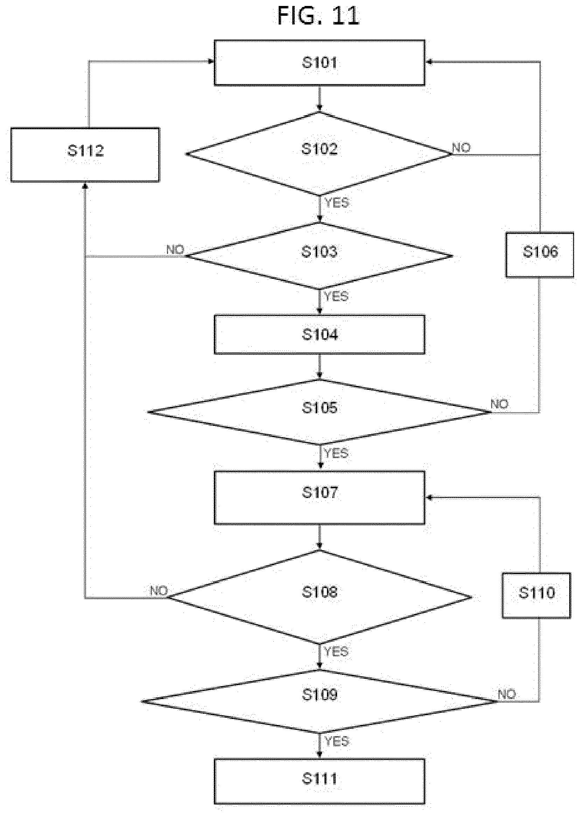

La FIG. 11 est un diagramme illustrant toutes les étapes requises pour la validation par l'unité de contrôle d'un code d'actions mécaniques combinées exécutées de façon prédéterminée par une personne. Lorsqu'un code prédéterminé est validé, l'unité de contrôle envoie une commande correspondante au dispositif implantable.

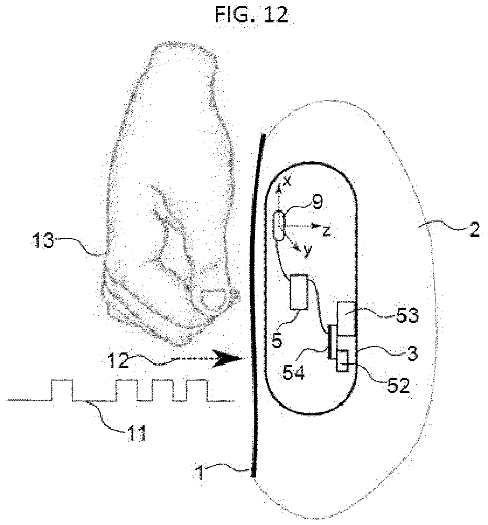

La FIG. 12 montre une configuration du système de détection des actions mécaniques exécutées par une personne lorsque les capteurs sont un accéléromètre 3 axes alimenté par une source électrique et un capteur piézoélectrique qui ne demande pas d'alimentation électrique. Dans cette configuration, les actions mécaniques sont des accélérations, ou plus généralement des vibrations (par exemple contractions musculaires, mouvements abdominaux...) initiées volontairement par l'utilisateur. Lorsqu'une vibration est suffisamment élevée, au-dessus d'un seuil prédéterminé, un signal électrique est généré par le capteur piézoélectrique et envoyé à l'unité de contrôle afin de réveiller l'unité de contrôle et l'accéléromètre 3 axes, précédemment dans un mode de veille, afin d'initier la phase de détection.

### DESCRIPTION DÉTAILLÉE DES MODES DE RÉALISATION PRÉFÉRÉS

La présente invention est de préférence mise en oeuvre dans un dispositif implantable dans un corps humain ou animal dans le but de contrôler ce dernier sans l'aide d'aucun périphérique externe et dans le but d'offrir une solution ergonomique et sûre pour le contrôle du dispositif. L'utilisateur peut être la personne qui porte le dispositif implantable ou une tierce personne. Afin d'envoyer une commande au dispositif implantable, l'utilisateur doit exécuter un code prédéterminé d'actions mécaniques qui peuvent être distinguées par une unité de contrôle comme une commande spécifique parmi toutes les données mesurées par le(s) capteur(s) dans l'activité quotidienne de la personne qui porte le dispositif implantable.

La présente invention peut-être être employée dans diverses applications. A des fins d'illustration, la présente invention sera décrite dans le cadre des dispositifs médicaux implantables (DMI) tels que les stimulateurs cardiaques, défibrillateurs, neuromodulateurs, sphincters artificiels, anneaux gastriques, implants orthopédiques ou pompes à infusion.

De nombreuses fonctions d'un DMI peuvent être contrôlées en utilisant la présente invention. Le DMI peut être activé ou désactivé entièrement (c'est-à-dire respectivement éteint ou allumé) ou partiellement (c'est-à-dire que seulement certaines fonctions du DMI sont activées). Ce contrôle peut trouver une utilité importante dans des DMI tels que les neuromodulateurs, pour lesquels la thérapie peut être temporairement arrêtée par le patient afin d'augmenter la durée de vie du DMI, en réduisant la consommation d'énergie lorsque le traitement n'est pas nécessaire. Par exemple, dans le cas de la neurostimulation de la racine sacrée S3, le patient pourrait désactiver le DMI sans aucun périphérique externe de contrôle.

La présente invention peut également être employée afin d'offrir au personnel médical une désactivation du DMI en cas d'urgence.

La présente invention peut également être employée dans un DMI pour lequel la thérapie peut être ajustée.

Le sphincter artificiel est une application particulière intéressante d'un DMI pour lequel la présente invention peut-être être employée. Dans le cas d'un sphincter urinaire artificiel, pour déclencher la miction (c'est-à-dire à ouvrir une manchette occlusive implantée), le patient exécute un code prédéterminé d'actions mécaniques. Un autre code peut être utilisé pour arrêter la miction (c'est-à-dire fermer la manchette occlusive). Plus généralement, la présente invention peut être utilisée pour modifier la forme du DMI (dans le cas par exemple des anneaux gastriques ou des prothèses artificielles), pour contrôler les fonctions de sécurité du DMI, telles que l'arrêt d'urgence ou une thérapie d'urgence, pour modifier un ou plusieurs paramètres du DMI, tels que les paramètres de thérapie, ou enfin pour réveiller ou mettre en veille une ou plusieurs fonctions du DMI.

Le DMI dans lequel la présente invention est implémentée est équipé d'un ou plusieurs capteurs appropriés capables de mesurer des séries d'actions mécaniques qui peuvent être de préférence des pressions, chocs, accélérations, mouvements, vibrations ou contractions musculaires. Les types de capteurs qui peuvent être utilisés sont de préférence des capteurs de pression, des commutateurs, des gyroscopes ou des accéléromètres comportant au moins un axe. Une autre méthode pour mesurer indirectement les actions mécaniques initiées par des contractions musculaires peut-être être l'électromyographie, qui est couramment utilisée pour mesurer l'activité musculaire. Enfin, les systèmes de localisation (système de localisation locale ou GPS) peuvent servir à mesurer les variations de position relatives aux actions mécaniques.

Afin de mettre en oeuvre la présente invention, une unité de contrôle doit être utilisée pour l'acquisition, le conditionnement, le stockage et le traitement des données provenant du ou des capteurs. Cette unité de contrôle peut être spécialement dédiée à la présente invention ou peut être incluse dans l'unité de contrôle du DMI, gérant les données provenant du ou des capteur(s), mentionnée plus haut.

Les FIG. 1 à FIG. 7 illustrent des configurations matérielles préférées pour la mise en oeuvre de la présente invention. Diverses configurations matérielles sont possibles selon le site d'implantation du DMI et selon le(s) capteur(s) utilisé(s) pour mesurer les actions mécaniques. En outre, en fonction de la nature des actions mécaniques, les parties implantables (capteur(s), unité de contrôle et DMI) doivent être placés de manière à maximiser la qualité de détection de la présente invention.

A des fins d'illustration, un seul capteur est représenté sur les FIG. 1 à FIG. 7. Il doit être entendu que plus d'un capteur peuvent être implémentés avec une configuration matérielle de chaque capteur de préférence telle que décrite sur l'une des figures FIG. 1 à FIG. 7.

Dans la FIG. 1, le capteur **4** et l'unité de contrôle **5** sont intégrés au DMI **3,** qui est implanté à l'intérieur d'une cavité du corps **2** sous la peau **1.** Dans cette configuration la nature des actions mécaniques est de préférence des chocs, vibrations ou accélérations générés soit sur la peau du patient, soit par des contractions musculaires. En ce qui concerne l'unité de contrôle, elle peut être l'unité de contrôle du DMI, gérant les données provenant du ou des capteurs, mentionné plus haut.

Dans la FIG. 2, le capteur **4** et l'unité de contrôle **5** sont intégrés à l'intérieur d'un boîtier implantable **7** afin d'être placé à l'intérieur d'une partie du corps qui est par exemple mal adaptée pour l'implantation du DMI lui-même. Dans ce cas, l'unité de contrôle communique via une communication filaire ou sans fil **6** avec le DMI. La communication est bidirectionnelle afin d'envoyer et de recevoir des données dans les deux terminaux. Dans cette configuration matérielle, n'importe quel type d'action mécanique peut être mesuré, selon le capteur utilisé.

La FIG. 3 illustre la même configuration, excepté que l'unité de contrôle **5** est intégrée à l'intérieur du DMI **3.** Cette configuration peut être utile si l'espace où le capteur **4** est implanté est restreint. Le capteur comporte un boîtier implantable et communique avec l'unité de contrôle **5** placée à l'intérieur du DMI **3** grâce à une communication filaire ou sans fil **8.** L'unité de contrôle peut être celle du DMI, gérant les données provenant du capteur pour la détection des actions mécaniques exécutées dans un code prédéterminé par l'utilisateur.

La description qui suit présente des réalisations préférées mais non limitatives de l'invention. Les capteurs sont définis ci-dessus, mais ne sont pas limités à ceux cités. Ils peuvent être remplacés par n'importe quel capteur capable de mesurer le même type d'action mécanique.

Les configurations matérielles représentées dans les FIG. 4 à FIG. 7 sont illustrées avec le capteur et l'unité de contrôle intégrés au DMI. Il doit être entendu que les configurations matérielles mentionnées ci-dessus et représentées dans la FIG. 2 et la FIG. 3 peuvent être employées.

Une configuration préférée est décrite à partir des FIG. 4 et FIG. 5.

Le capteur dans cette configuration matérielle est un accéléromètre 3 axes **9** mesurant les accélérations du DMI **3.**

Un code prédéterminé **10** de chocs ou de vibrations est illustré sur la FIG. 4. Il se compose d'un silence, suivi par une action mécanique puis un nouveau silence suivi de trois actions mécaniques séparées par de courts silences. Dans cette configuration, l'action mécanique peut être des contractions musculaires volontaires induisant des vibrations ou des chocs à l'intérieur de la cavité du corps **2** où se trouve le DMI **3.** Sur la FIG. 5, le choc, mesuré par l'accéléromètre 3 axes pour la détection du code prédéterminé d'actions mécaniques **11,** est effectué à l'extérieur du corps. L'utilisateur exécute avec sa main **13** le code prédéterminé en tapotant sur la peau **1** (ou en tout état de cause, au-dessus du site d'implantation du DMI comportant le capteur), dans la direction **12** du DMI **3** et où se trouve le DMI. Le code **11** représenté sur la FIG. 5 correspond à un silence, suivi par le tapotement de la main sur la peau **1,** puis un nouveau silence suivi par trois tapotements sur la peau, séparés par de courts silences. Dans les deux cas illustrés par la FIG. 4 et la FIG. 5, les signaux envoyés à l'unité de contrôle sont les trois signaux venant des mesures d'accélération des axes X, Y et Z de l'accéléromètre afin d'avoir une signature la plus précise possible de l'action mécanique.

Il doit être compris qu'un accéléromètre avec seulement un ou deux axes de mesure peut être utilisé si la signature de l'action mécanique est suffisamment précise avec un ou deux signaux provenant de l'accéléromètre.

Dans le cas de l'accéléromètre, les silences peuvent être définis par une accélération dynamique ou un paramètre de l'accélération dynamique en dessous d'un seuil prédéterminé qui peut être combinée à une position spécifique. Par exemple, dans le cas d'un sphincter urinaire artificiel implantable, la miction peut être contrôlée par le patient en utilisant la présente invention. Pour déclencher l'ouverture du sphincter urinaire artificiel, le patient devra tapoter avec sa main sur la peau où le capteur du sphincter artificiel est situé comme décrit ci-dessus. Dans ce cas, les silences correspondent au patient maintenant, en position quasi-immobile, son buste en position verticale. Dans cette posture, et quand le patient ne bouge pas, les signaux de sortie, provenant d'un accéléromètre micro-fabriqués standard comportant une masselotte suspendue par des ressorts (par exemple, le capteur ADXL335 commercialisé par la société Analog Devices), ont une composante AC (correspondant à l'accélération « dynamique ») d'un faible niveau. La composante DC (correspondant à l'accélération « statique ») de chaque axe de mesure peut servir à définir la posture du patient grâce à la mesure de la gravité terrestre.

Les FIG. 6 et FIG. 7 représentent une configuration matérielle conçue pour mesurer la pression sur le boîtier du DMI (ou le boîtier de l'unité de détection s'il est déporté du DMI). Dans une réalisation privilégiée, le capteur **15, 17** est un capteur de pression ou un commutateur pour lesquels le signal de sortie varie en fonction de la pression appliquée sur le capteur. Il peut être aussi un capteur hydraulique ou pneumatique relié à un ballon, générant une pression lorsque le ballon est déformé. Dans la FIG. 6, le capteur **15** est placé de façon à ce que la pression appliquée sur la peau **1** peut être mesurée. La pression est appliquée dans la direction représentée par la flèche **16.** Un code prédéterminé **14** composée de pressions exercées sur la peau (par exemple avec la main de l'utilisateur), combinée avec des silences est détecté par le capteur **15** et l'unité de contrôle **5.** Les silences dans cette configuration sont définis comme le signal de sortie du capteur ou un paramètre du signal de sortie sous un seuil prédéterminé (basse pression appliquée sur la peau). La même configuration est illustrée sur la FIG. 6, sauf que, dans ce cas, le capteur **17** est placé de sorte à ce que la pression soit mesurée à l'intérieur de la cavité du corps **2.** La pression est mesurée principalement dans le sens représenté par la flèche **19.** Dans ce cas, la pression peut être générée par les contractions des muscles qui entourent l'espace **2,** où se trouve le DMI **3.** Le code prédéterminé **18** respecte les mêmes contraintes du code **14** définie ci-dessus et représenté sur la FIG. 6.

Une combinaison de capteurs pour mesurer des actions mécaniques ou des silences de natures différentes peuvent être également implémentées. Par exemple, dans le cas de tapotements sur la peau, le dispositif peut implémenter la combinaison de mesure d'accélérations (mesurées par un accéléromètre) et de pressions (mesurées par un capteur de pression sous-cutané).

Les configurations matérielles privilégiées ont été décrites dans les paragraphes ci-dessus.

Il est important de définir une bonne configuration matérielle afin d'avoir, selon l'action mécanique qui est employée, la meilleure mesure et donc la possibilité de recueillir des données précises.

Dans les paragraphes suivants, la procédure et la méthode permettant l'acquisition et le traitement des données recueillies sont décrites.

Sans une acquisition et une méthode de traitement adéquates, le code prédéterminé d'actions mécaniques ne peut être détecté à chaque fois qu'il est exécuté par l'utilisateur. D'un autre côté, la méthode doit être suffisamment précise pour fournir une solution fiable permettant de détecter les codes seulement lorsqu'ils sont exécutés, mais pas par erreur à cause de signaux parasites (par exemple, les mouvements intempestifs du patient, la respiration, des pressions appliquées sur la peau non désirées ou des contractions musculaires), mesurés par le capteur et traités par l'unité de contrôle. Afin d'offrir une solution fiable et pour éviter de fausses détections d'un code, ces aspects sont pris en compte dans la présente invention et sont décrits dans les paragraphes suivants.

Comme exposé précédemment, le code prédéterminé est constitué d'actions mécaniques combinées à au moins une information supplémentaire détectable par au moins un capteur, par exemple des périodes de silences. Les silences sont définis en fonction du capteur utilisé comme un ou des signaux de sortie traités avec un niveau de faible amplitude. Un seuil est utilisé pour déterminer que le ou les signaux de sortie traités et conditionnés par l'unité de contrôle ont un niveau assez faible et correspondent bien à un silence. Dans certaines conditions, d'autres informations telles que la position ou la posture du patient qui porte le DMI implanté, ou bien un paramètre spécifique du silence peuvent servir afin d'augmenter la fiabilité de la détection.

La configuration matérielle représentée sur la FIG. 5 est employée dans le présent paragraphe à des fins d'illustration. Seules les accélérations sur l'axe Z de l'accéléromètre **9** sont mesurées. Le graphique représenté sur la FIG. 8 illustre le signal de sortie de l'accéléromètre correspondant à la mesure de l'accélération le long de l'axe des Z (suivant la direction représentée par la flèche **12**). Le signal de sortie est traité ; il est filtré par un filtre passe-bande de manière à récupérer seulement le signal AC de l'accélération (c'est-à-dire la composante dynamique) et à supprimer le bruit dans les hautes fréquences, puis rectifié pour enfin extraire l'enveloppe. La composante DC, non représentée sur la FIG. 8 peut également être utilisée afin de connaître approximativement la position du patient (environ 0 g lorsque le patient a son buste dans une posture verticale et autour de ±1 g quand il a son buste dans une position horizontale si le DMI est situé dans cette région du corps humain). Les formes d'ondes **24, 29, 33** et **38** correspondent au signal mesuré par le capteur et traité par l'unité de contrôle lorsque l'utilisateur exécute un tapotement manuel sur la peau, sur le site d'implantation du dispositif. Les périodes de silence correspondent dans cette configuration à un signal de sortie traité (ou bien un des paramètres du signal) qui a un niveau au-dessous du seuil de **20.** En outre, dans certaines applications, il peut être possible de rendre la détection du code plus robuste et fiable en utilisant le signal de sortie DC de l'accéléromètre afin d'avoir une estimation de la posture du patient. Les fenêtres de temps **23, 28, 35** et **40,** combinés avec les silences, représentent un code typique exécuté, respectant des délais requis. Les tolérances des durées sont appliquées sur la durée des actions mécaniques ainsi que sur la durée des silences. Lorsqu'une action mécanique est détectée, dans le cas illustré, lorsque le signal traité est au-dessus du seuil **21,** les actions mécaniques exécutées dans les fenêtres de temps **23, 28, 35** et **40,** doivent respectivement se terminer (niveau de signal au-dessous du seuil **20**) dans la fenêtre de temps **25, 30, 34 et 39** afin d'être validés. Le code présenté dans la FIG. 8 est composé de quatre actions mécaniques (tapotement de la main) et de cinq périodes de silence. Afin de valider le code, l'utilisateur doit respecter un délai minimum de silence **22** suivie d'une action mécanique, puis une nouvelle période de silence, suivi par trois actions mécaniques, séparées par un court laps de temps de silence. Enfin, à la fin de la dernière action mécanique, un délai minimum de silence **41** doit être respecté. Au cours de la période d'exécution du code, l'utilisateur ne bouge pas la partie du corps où le DMI est implanté. Des contraintes de temps sont également définies pour chaque période de silence. Dans le cas de la première et de la dernière période de silence, une contrainte de temps minimale doit être respectée. Concernant les périodes de silence qui séparent les périodes d'actions mécaniques, des périodes minimales prédéterminées, **26, 31, 36** sont également définies. Une autre contrainte durant les silences concerne des périodes de temps maximum **27, 32, 37** à respecter. Une période maximale de temps peut également être définie pour le premier silence en fonction de l'application ciblée.

Pour chaque commande, les contraintes de durée, silences et nombre d'actions mécaniques mentionnées ci-dessus sont définies afin de déterminer un code spécifique et prédéterminé. Les actions mécaniques ne sont pas nécessairement les mêmes dans le même code. Tous les paramètres peuvent être ajustés par le médecin afin de convenir à chaque patient. Cela peut être réalisé à partir d'un paramétrage via la communication sans fil entre le DMI et une base externe. Les deux terminaux doivent pouvoir gérer une communication bidirectionnelle afin de paramétrer le dispositif implantable correctement.

Afin d'avoir une méthode de détection robuste et fiable, les codes prédéterminés d'actions mécaniques ne peuvent pas être simplement détectés à l'aide d'un simple dépassement de seuil du niveau d'amplitude du signal et des contraintes de temps à respecter. En effet, le système mesure les actions mécaniques parmi les activités quotidiennes du patient. La procédure de détection de code doit par conséquent être très robuste afin d'éviter une fausse détection d'un code en mesurant un motif d'actions mécaniques qui, après traitement du signal, aurait les mêmes caractéristiques en terme de délais et de niveau amplitude, comme présenté plus haut, du code prédéterminé. Ainsi, parmi toutes les données recueillies par le système de mesure tout au long de la durée de vie du dispositif implantable, les codes effectivement exécutés par l'utilisateur doivent être identifiés précisément et uniquement dans cette situation. En outre, si la présente invention est utilisée dans des applications de DMI, les fausses détections ou les non-détections liées à certaines commandes, telles que l'arrêt d'urgence ou la modification du traitement peuvent être critiques pour le patient. Il est donc très important de fournir une solution fiable permettant la détection d'un code seulement et uniquement lorsque celui-ci est exécuté correctement.

En plus de la procédure d'acquisition décrite en référence à la FIG. 8, la méthode comprend également une procédure de validation du code prédéterminé.

La FIG. 11 représente un diagramme bloc illustrant les étapes qui peuvent être exécutés avant la validation d'une commande.

La nomenclature des blocs, dont le contenu est exposé en détail ci-dessous est la suivante :
S101 : Acquisition données capteur(s)
S102 : Action mécanique ou silence détecté ?
S103 : Délais prédéterminés respectés ?
S104 : Mémorisation des jeux de données i
S105 : i = nombre d'actions mécaniques et de silences à détecter ?
S106 i = i+1
S107 : Comparaison du jeu de données j à un modèle de référence
S108 : Correspondance du jeu de données j avec le modèle de référence ?
S109 : j = nombre d'actions mécaniques et de silences à valider ?
S110 : j = j+1
S111 : Envoi au dispositif implantable de l'ordre associé à la commande
S112 : Suppression des jeux de données mémorisés s'il y en a, i=0, j=0.

Les étapes **S101** à **S106** et **S112** correspondent à la procédure décrite ci-dessus et illustrée par la FIG. 8. C'est la première étape de la méthode. Au cours de l'étape **S101,** les données sont mesurées en continu, par période ou en utilisant des méthodes adaptatives qui offrent la possibilité de mesurer les données uniquement lorsque cela est nécessaire (par exemple, échantillonneur non uniforme) afin de minimiser la consommation d'énergie du DMI. L'indice i correspond au nombre d'actions mécaniques et de silences à être détecter dans le code. Si plusieurs codes prédéterminés sont programmés, le nombre maximal d'index i peut être modifié par l'unité de contrôle au cours de la procédure de détection en fonction des délais qui doivent être respectés pour chaque code. En d'autres termes, au cours de l'étape **S103,** les durées des actions mécaniques et des silences enregistrées dans la mémoire selon l'indice i (en fonction des dernières actions mécaniques et silences déjà exécutés) sont comparées au dernier événement mesuré afin de savoir quel code programmé en mémoire peut être potentiellement pris en compte. Si la durée ne correspond pas à une des périodes de temps relatives à une action mécanique ou à un silence listés en mémoire, le système passe à l'étape **S112,** sinon l'index est incrémenté (étape **S106**) et les références de codes prédéterminés qui peuvent potentiellement être ceux qui peuvent être détectés sont stockés en mémoire. La première procédure de la méthode est répétée jusqu'à ce qu'un code soit détecté. La procédure de validation est réalisée dans une deuxième étape, représentée par les étapes **S107** à **S112.** L'unité de contrôle applique, pour chaque jeu de données enregistré dans la première étape, correspondant à une action mécanique ou un silence (information supplémentaire), une comparaison avec un modèle de référence associé, stocké dans la mémoire. La procédure de validation est répétée jusqu'à ce que l'indice j atteigne le nombre de comparaisons à réaliser. La procédure peut être arrêtée à tout moment si un jeu de données enregistré ne correspond pas à son modèle de référence potentiellement associés. Dans ce cas, l'étape **S112** est exécutée. La mémorisation de signaux n'est pas obligatoire pour les actions mécaniques ou les silences. La comparaison peut effectivement se faire sur des signaux en temps réel afin d'éviter un stockage excessif de données en mémoire.

Pour des questions d'économie d'énergie, la comparaison des données est effectuée individuellement, pour chaque action mécanique, afin de réduire les données à traiter et ainsi les ressources de calcul et de temps requis par l'unité de contrôle pour la comparaison mathématique. Cependant, le modèle de référence stocké dans la mémoire peut correspondre au motif complet de données d'un code prédéterminé d'actions mécaniques. Dans ce cas, l'indice j dans le diagramme de bloc de la FIG. 11 n'est pas utilisé, et la seconde procédure est exécutée une fois.

La FIG. 9 illustre un exemple de comparaison de signaux d'une action mécanique. Les signaux illustrés sont des signaux de sortie qui ont été traités et conditionnés, mesurés par un accéléromètre selon la configuration matérielle représentée dans la FIG. 5 le long de l'axe Z. Le signal est l'enveloppe du signal brut filtré par un passe-bande et rectifié. Dans l'exemple, la comparaison est effectuée sur la base des paramètres de la forme du signal. Le modèle de référence **42** du signal stocké dans la mémoire est comparé dans un premier cas au signal mesuré, traité et conditionné **43** précédemment enregistré. On peut ainsi déterminer un indice de similarité entre le signal de commande et le signal de référence. Les paramètres de la forme des deux signaux correspondent dans ce cas (i.e. l'indice de similarité est supérieur à une valeur seuil déterminée) (notation OK) ; le signal mesuré **43** est validé dans les étapes **S107** et **S108** de la procédure de détection (FIG. 11). Dans le second cas, le modèle de référence du signal **42** est comparé avec le signal mesuré, traité et conditionné **44** précédemment enregistré. Dans ce cas, les caractéristiques relatives à la forme du signal ne correspondent pas (i.e. l'indice de similarité est inférieur à une valeur seuil déterminée) (notation NOK). L'étape **S107** n'est pas validée. La détection est abandonnée et les données mesurées ainsi que les paramètres liés à cette détection sont effacées dans l'étape **S112** et la procédure est répétée à partir de l'étape **S101.**

Les fonctions de comparaison sont mises en oeuvre dans l'unité de contrôle. Ce sont des fonctions mathématiques qui permettent de valider les similitudes de plusieurs jeux de données. Dans les modes de réalisation préférés, les formes globales des signaux sont comparées grâce à des fonctions mathématiques telles que la méthode des moindres carrés en valeurs normalisés ou des fonctions d'inter-corrélation normalisée qui prennent en compte la forme des signaux pour effectuer la comparaison. Des méthodes telles que la déformation temporelle dynamique (plus connu sont le nom anglo-saxon « dynamique time warping ») peuvent aussi servir pour offrir une tolérance sur les durées entre le modèle de référence et le signal à comparer. En effet, si le signal est dilaté dans le temps ou comprimé, la méthode de déformation temporelle dynamique est capable de comparer la forme globale du signal même si cette forme est dilatée ou comprimée dans le temps par rapport au modèle de référence. Dans un autre mode de réalisation préféré, la comparaison peut s'effectuer sur des caractéristiques autres que les paramètres de forme du signal. Les caractéristiques de fréquence ou de temps, combinés ou pas avec des caractéristiques d'amplitude, de données statistiques ou de forme du signal peuvent également être employées pour procéder à la comparaison d'un motif de signaux prétraité à un modèle de référence préenregistrés de caractéristiques du signal. Il doit être entendu que l'objectif de cette phase, décrite dans la FIG. 11 par les étapes **S107** et **S108** consiste à déterminer si la signature du signal mesuré, traité et enregistré dans la mémoire, correspond à la signature réelle de référence préenregistrée en mémoire afin de savoir si elle correspond à l'action mécanique attendue (ou bien à un silence attendu, le cas échéant).

Il faut noter que pendant la phase de détection, le système peut toujours effectuer des mesures à partir des capteurs.

Il est important de noter que le cas présenté ci-dessus n'est qu'un exemple. Il peut être différent, tant que la méthode se compose de la détection d'un code d'actions mécaniques avec un rythme prédéterminé et d'un nombre d'actions prédéterminées, combiné avec l'identification de chaque signature d'action mécanique et d'information supplémentaire dans le but de valider la commande exécutée par l'utilisateur.

Dans la FIG. 10, les fonctions matérielles nécessaires à la mise en oeuvre de la méthode de détection décrite dans la présente invention sont présentées. Dans la FIG. 10, l'unité de contrôle est l'unité de contrôle **51** du dispositif implantable. L'interface capteur **46** permet d'acquérir les données provenant de l'unité de mesure **45** qui peut être composée de plusieurs capteurs. Le bloc **49** est l'unité de traitement. Elle peut par exemple être un contrôleur numérique ou un microprocesseur. Sa fonction est d'effectuer les traitements du signal, les traitements mathématiques, la communication avec les autres blocs et le traitement des données. Des horloges **48** et une mémoire **47** sont également utilisés pour le traitement des données. Lorsqu'un code est validé, l'unité de traitement **49** envoie l'ordre associé à une fonction dédiée incluse dans le bloc **50.**

Le dispositif peut être équipé d'un système de télémétrie afin de permettre une configuration de la méthode de détection. La sensibilité, les tolérances, les codes prédéterminés et les ordres associés peuvent être des paramètres qui peuvent être configurés avec le système sans fil externe, communiquant avec l'unité de contrôle. Lorsqu'une commande est validée, un retour tel que des vibrations ou un signal sonore peut être généré par le dispositif implantable afin d'avertir le patient ou une tierce personne de la validation d'une commande.

Afin d'annuler une commande, après l'exécution du code à annuler, l'utilisateur peut effectuer des actions mécaniques associées au code plusieurs fois rapidement. Par exemple, la présente invention peut-être être employée dans un sphincter urinaire artificiel équipé d'un accéléromètre pour mesurer des tapotements manuels de contrôle sur l'abdomen. Afin d'éviter la miction après avoir exécuté un code prédéterminé, le patient peut exécuter plusieurs tapotements plusieurs fois rapidement sur l'abdomen, où se trouve l'implant, pour envoyer une commande à l'unité de contrôle de fermeture instantanée de la manchette occlusive.

L'une des principales contraintes dans les dispositifs implantables actifs est la consommation d'énergie. Afin d'éviter d'alimenter continuellement le ou les capteurs, l'un des capteurs ou le capteur peut être un dispositif qui permet de générer un signal électrique sans avoir besoin d'être alimenté par une source électrique. Par exemple, un capteur peut être conçu avec des matériaux piézoélectriques ou magnétiques afin de générer un signal électrique lorsqu'une action mécanique est appliquée sur le capteur. La FIG. 12 illustre une configuration préférée, mais non limitée, comprenant un accéléromètre 3 axes **9** et un capteur de vibration piézoélectrique. Le capteur piézoélectrique est constitué d'une masse sismique **52** suspendu par une structure souple **54** solidaire d'une structure rigide **53,** solidaire elle-même du dispositif implantable. Une couche piézoélectrique sur la structure souple **54** est utilisée pour générer un signal électrique lorsque la structure souple est fléchie par les mouvements de la masse sismiques générés par les vibrations externes. Le signal électrique généré est ensuite utilisé pour réveiller l'unité de contrôle **5** (par exemple en utilisant une broche d'interruption) et l'accéléromètre 3 axes, tous deux encore dans un mode veille afin de minimiser la consommation électrique. La procédure de détection d'un code prédéterminé d'actions mécaniques est ensuite traitée tel que décrit dans la présente invention. Lorsque la phase de détection est effectuée, l'unité de contrôle et l'accéléromètre retournent en mode veille.

Un dispositif externe peut être utilisé afin de générer le code prédéterminé d'actions mécaniques. Par exemple, un téléphone mobile avec une application contrôlant le vibreur peut générer une vibration sur la peau du patient, dans la zone d'implantation. Dans le cas de la détection par un accéléromètre, le code fait de vibrations et de silences peut être alors détecté par le système implanté dans le corps du patient.

## Revendications

1. Système de contrôle d'un dispositif (3) implanté dans un corps humain ou animal, à partir d'ordres exécutés par un utilisateur, comprenant :
- au moins un capteur (4) adapté pour mesurer des actions mécaniques exercées de manière volontaire par l'utilisateur sur ou dans le corps dans lequel est implanté le dispositif (3),
- une unité de contrôle (5) adaptée pour :
• le traitement d'au moins un signal venant dudit capteur (4),
• la détection, dans ledit signal, d'un code prédéterminé exécuté par l'utilisateur, selon un motif comprenant au moins une action mécanique et au moins une information supplémentaire détectable par le(les)dit(s) capteur(s) (4), ladite détection comprenant :
∘ la détection de ladite information supplémentaire, dans une portion dudit signal qui a un niveau inférieur à un seuil (20) prédéterminé durant une période présentant une durée comprise entre une durée minimale et une durée maximale,
∘ la détection de ladite action mécanique dans une portion dudit signal qui a un niveau au-dessus d'un seuil (21) prédéterminé durant une période présentant une durée comprise entre une durée minimale et une durée maximale d'action mécanique après une information supplémentaire,
• l'identification de chaque action mécanique et chaque information supplémentaire mesurées dans une portion dudit signal traité,
• la comparaison des caractéristiques de chaque portion dudit signal correspondant à une action mécanique avec un modèle de référence des caractéristiques du signal correspondant, l'unité de contrôle comprenant une mémoire dans laquelle est stocké au moins un modèle de référence des caractéristiques dudit signal, ladite comparaison comprenant l'évaluation d'un indice de similarité entre le signal traité et le signal de référence,
• la détermination, à partir de la dite comparaison, si ladite au moins une mesure d'action mécanique et ladite au moins une information supplémentaire appartiennent effectivement au dit code prédéterminé et
• l'envoi d'un ordre prédéterminé au dit dispositif (3) implanté si le motif appliqué correspond au code prédéterminé,
ledit capteur (4) et ladite unité de contrôle (5) étant implantables dans ledit corps animal ou humain.

2. Système selon la revendication 1, **caractérisé en ce que** le(les)dit(s) capteur(s) et ladite unité de contrôle sont adaptés pour la détection d'une posture prédéterminée dudit corps humain ou animal pendant une durée prédéterminée, correspondant à ladite information supplémentaire.

3. Système selon l'une des revendications 1 à 2, **caractérisé en ce que** ladite unité de contrôle comprend des moyens pour la mesure et la détection de la durée de chaque information supplémentaire détectable et de chaque action mécanique.

4. Système selon l'une des revendications 1 à 3, **caractérisé en ce que** ladite unité de contrôle comprend des moyens pour effectuer en outre les étapes suivantes :
a) détection de ladite information supplémentaire, dans une portion dudit signal qui a un niveau inférieur à un seuil prédéterminé durant une période présentant une durée comprise entre une durée minimale et une durée maximale,
b) détection de ladite action mécanique dans une portion dudit signal qui a un niveau au-dessus d'un seuil prédéterminé durant une période présentant une durée comprise entre une durée minimale et une durée maximale d'action mécanique après une information supplémentaire,
c) stockage des caractéristiques dudit signal dans une mémoire,
d) répétition des étapes (a) à (c) pour chaque portion dudit signal jusqu'à la fin du motif,
e) si ledit motif ne correspond pas audit code prédéterminé, réinitialisation à l'étape (a) à tout moment jusqu'à la détection d'un motif correspondant au dit code prédéterminé.

5. Système selon l'une des revendications 1 à 4, **caractérisé en ce que** ledit ordre prédéterminé à envoyer au dispositif implanté est choisi parmi une activation ou une désactivation dudit dispositif implanté, une activation ou une désactivation d'au moins une fonction dudit dispositif implanté, une modification d'au moins un paramètre dudit dispositif implanté, une modification d'une thérapie appliquée par ledit dispositif implanté, une modification de la forme dudit dispositif implanté et une activation ou une désactivation d'une fonctionnalité d'au moins une sécurité dudit dispositif implanté.

6. Système selon l'une des revendications 1 à 5, **caractérisé en ce qu'**il comprend des moyens pour générer une rétroaction pour l'utilisateur lorsque ledit ordre prédéterminé est envoyé audit dispositif implanté.

7. Système selon l'une des revendications 1 à 6, **caractérisé en ce que** le(les)dit(s) capteur(s) est(sont) adapté(s) pour la mesure d'une action mécanique choisie parmi une percussion manuelle extérieure sur le corps, au niveau du site d'implantation dudit capteur, une contraction musculaire, des vibrations, une augmentation de la pression d'une cavité intracorporelle et une augmentation de la pression sur la peau au-dessus du site d'implantation dudit capteur.

8. Système selon l'une des revendications 1 à 7, **caractérisé en ce que** ledit dispositif implanté contrôlé est un sphincter artificiel et **en ce que** ledit ordre prédéterminé comprend l'ouverture ou la fermeture d'un élément occlusif dudit sphincter artificiel.

9. Système selon l'une des revendications 1 à 8, **caractérisé en ce qu'**il comprend des moyens pour mettre à jour périodiquement le(s)dit(s) modèle(s) de référence.

10. Système selon l'une des revendications 1 à 9, **caractérisé en ce qu'**au moins deux codes prédéterminés différents sont stockés dans ladite mémoire, chacun de ces codes, lorsqu'il est appliqué sur le corps, conduisant à l'envoi au dispositif implanté d'un ordre respectif spécifique.

11. Système selon l'une des revendications 1 à 10, comprenant en outre un système de communication sans fil entre le dispositif implantable et une base sans fil externe adaptée pour configurer les paramètres de détection des codes prédéterminés.

12. Système selon l'une des revendications 1 à 11, **caractérisé en ce que** ledit au moins un capteur est choisi parmi un accéléromètre 1, 2 ou 3 axes, un gyroscope, un système de localisation, un capteur de pression et un commutateur électrique.

13. Système selon l'une des revendications 1 à 12, dans lequel ledit capteur est un capteur dépourvu d'alimentation électrique, générant au moins un signal électrique lorsqu'une action mécanique est appliquée sur ledit capteur.

14. Dispositif implantable adapté pour être implanté dans un corps humain ou animal, comprenant un système de contrôle conformément à l'une des revendications 1 à 13.

15. Dispositif implantable selon la revendication 14, consistant en un sphincter artificiel.

## Patentansprüche

1. System zur Steuerung, basierend auf von einem Benutzer ausgegebenen Befehlen, einer in einen Körper eines Menschen oder eines Tieres implantierten Vorrichtung (3), umfassend:
- mindestens einen Sensor (4), der ausgebildet ist, um mechanische Aktionen zu messen, die absichtlich von dem Benutzer auf oder in dem Körper durchgeführt werden, in dem die Vorrichtung (3) implantiert ist,
- eine Steuereinheit (5), die ausgebildet ist für:
• die Verarbeitung mindestens eines Signals, das von dem Sensor (4) kommt,
• die Ermittlung, in dem Signal, eines vorbestimmten, von dem Benutzer ausgegebenen Codes, gemäß einem Motiv, das mindestens eine mechanische Aktion und mindestens eine zusätzliche Information umfasst, die von dem/n Sensor(en) (4) ermittelbar ist, wobei die Ermittlung umfasst:
∘ die Ermittlung der zusätzlichen Information in einem Abschnitt des Signals mit einem niedrigeren Niveau als ein vorbestimmter Grenzwert (20) während eines Zeitraums, der eine Dauer zwischen einer minimalen Dauer und einer maximalen Dauer inklusive aufweist,
∘ die Ermittlung der mechanischen Aktion in einem Abschnitt des Signals mit einem Niveau über einem vorbestimmten Grenzwert (21) während eines Zeitraums, der eine Dauer zwischen einer minimalen Dauer und einer maximalen Dauer inklusive einer mechanischen Aktion nach einer zusätzlichen Information aufweist,
• die Identifizierung jeder mechanischen Aktion und jeder zusätzlichen Information, die in einem Abschnitt des verarbeiteten Signals gemessen werden,
• den Vergleich der Merkmale jedes Abschnitts des Signals, das einer mechanischen Aktion entspricht, mit einem Referenzmodell der Merkmale des entsprechenden Signals, wobei die Steuereinheit einen Speicher umfasst, in dem mindestens ein Referenzmodell der Merkmale des Signals gespeichert ist, wobei der Vergleich die Bewertung eines Ähnlichkeitshinweises zwischen dem verarbeiteten Signal und dem Referenzsignal umfasst,
• die Bestimmung, auf der Basis des Vergleichs, ob die mindestens eine Messung einer mechanischen Aktion und die mindestens eine zusätzliche Information tatsächlich zu dem vorbestimmten Code gehören und
• das Senden eines vorbestimmten Befehls an die implantierte Vorrichtung (3), wenn das angewendete Motiv dem vorbestimmten Code entspricht,
wobei der Sensor (4) und die Steuereinheit (5) in dem Körper eines Tieres oder eines Menschen implantierbar sind.

2. System nach Anspruch 1, **dadurch gekennzeichnet, dass** der/die Sensor(en) und die Steuereinheit für die Ermittlung einer vorbestimmten Haltung des Körpers eines Menschen oder Tieres während einer vorbestimmten Dauer ausgebildet sind, die der zusätzlichen Information entspricht.

3. System nach einem der Ansprüche 1 bis 2, **dadurch gekennzeichnet, dass** die Steuereinheit Mittel für das Messen und die Ermittlung der Dauer jeder ermittelbaren zusätzlichen Information und jeder mechanischen Aktion umfasst.

4. System nach einem der Ansprüche 1 bis 3, **dadurch gekennzeichnet, dass** die Steuereinheit Mittel umfasst, um ferner die folgenden Schritte durchzuführen:
a) Ermitteln der zusätzlichen Information in einem Abschnitt des Signals mit einem niedrigeren Niveau als ein vorbestimmter Grenzwert während eines Zeitraums, der eine Dauer zwischen einer minimalen Dauer und einer maximalen Dauer inklusive aufweist,
b) Ermitteln der mechanischen Aktion in einem Abschnitt des Signals mit einem Niveau über einem vorbestimmten Grenzwert während eines Zeitraums, der eine Dauer zwischen einer minimalen Dauer und einer maximalen Dauer inklusive einer mechanischen Aktion nach einer zusätzlichen Information aufweist,
c) Speichern der Merkmale des Signals in einem Speicher,
d) Wiederholen der Schritte (a) bis (c) für jeden Abschnitt des Signals bis zum Ende des Motivs,
e) wenn das Motiv nicht dem vorbestimmten Code entspricht, Reinitialisieren in Schritt (a) jederzeit bis zur Ermittlung eines Motivs, das dem vorbestimmten Code entspricht.

5. System nach einem der Ansprüche 1 bis 4, **dadurch gekennzeichnet, dass** der an die implantierte Vorrichtung zu sendende vorbestimmte Befehl aus einer Aktivierung oder einer Deaktivierung der implantierten Vorrichtung, einer Aktivierung oder einer Deaktivierung mindestens einer Funktion der implantierten Vorrichtung, einer Änderung mindestens eines Parameters der implantierten Vorrichtung, einer Änderung einer von der implantierten Vorrichtung angewendeten Therapie, einer Änderung der Form der implantierten Vorrichtung und einer Aktivierung oder einer Deaktivierung einer Funktionalität mindestens einer Sicherheit der implantierten Vorrichtung ausgewählt ist.

6. System nach einem der Ansprüche 1 bis 5, **dadurch gekennzeichnet, dass** es Mittel umfasst, um eine Rückmeldung für den Benutzer zu erzeugen, wenn der vorbestimmte Befehl an die implantierte Vorrichtung gesendet ist.

7. System nach einem der Ansprüche 1 bis 6, **dadurch gekennzeichnet, dass** der/die Sensor(en) für die Messung einer mechanischen Aktion, ausgewählt aus einem äußeren manuellen Schlag auf den Körper im Bereich der Implantationsstelle des Sensors, einer Muskelkontraktion, Vibrationen, einer Erhöhung des Drucks eines Hohlraums im Körper und einer Erhöhung des Drucks auf die Haut oberhalb der Implantationsstelle des Sensors ausgebildet ist/sind.

8. System nach einem der Ansprüche 1 bis 7, **dadurch gekennzeichnet, dass** die gesteuerte implantierte Vorrichtung ein künstlicher Schließmuskel ist und dass der vorbestimmte Befehl das Öffnen oder das Schließen eines Verschlusselements des künstlichen Schließmuskels umfasst.

9. System nach einem der Ansprüche 1 bis 8, **dadurch gekennzeichnet, dass** es Mittel für die periodische Aktualisierung des/r Referenzmodells/-modelle umfasst.

10. System nach einem der Ansprüche 1 bis 9, **dadurch gekennzeichnet, dass** mindestens zwei unterschiedliche vorbestimmte Codes in dem Speicher gespeichert sind, wobei jeder dieser Codes, wenn er auf den Körper angewendet wird, zum Senden eines jeweiligen speziellen Befehls an die implantierte Vorrichtung führt.

11. System nach einem der Ansprüche 1 bis 10, umfassend ferner ein drahtloses Kommunikationssystem zwischen der implantierbaren Vorrichtung und einer externen drahtlosen Basis, die ausgebildet ist, um die Ermittlungsparameter der vorbestimmten Codes zu konfigurieren.

12. System nach einem der Ansprüche 1 bis 11, **dadurch gekennzeichnet, dass** der mindestens eine Sensor aus einem Beschleunigungsmesser mit 1, 2 oder 3 Achsen, einem Gyroskop, einem Lokalisierungssystem, einem Drucksensor und einem elektrischen Schalter ausgewählt ist.

13. System nach einem der Ansprüche 1 bis 12, wobei der Sensor ein Sensor ohne Stromversorgung ist, der mindestens ein elektrisches Signal erzeugt, wenn eine mechanische Aktion auf den Sensor ausgeübt wird.

14. Implantierbare Vorrichtung, die ausgebildet ist, um im Körper eines Menschen oder Tieres implantiert zu sein, umfassend ein Steuersystem nach einem der Ansprüche 1 bis 13.

15. Implantierbare Vorrichtung nach Anspruch 14, bestehend aus einem künstlichen Schließmuskel.

## Claims

1. A system for controlling a device (3) implanted in an animal or human body, on the basis of commands issued by a user comprising:
- at least one sensor (4) adapted for measuring mechanical actions exerted voluntarily by the user on or in the body in which the device (3) is implanted,
- a control unit (5) adapted for:
• processing at least one signal coming from said sensor (4),
• detecting a predetermined code issued by the user in said signal, according to a pattern comprising at least one mechanical action and at least one supplemental information detectable by said sensor (s) (4), said detection comprising:
∘ detecting said supplemental information in a portion of said signal that has a level below a predetermined threshold (20) during a period having a duration comprised between a minimum and a maximum duration,
∘ detecting said mechanical action in a portion of said signal that has a level above a predetermined threshold (21) during a period having a duration comprised between a minimum and a maximum mechanical action duration after a supplemental information,
• identifying each mechanical action and each supplemental information measured in a portion of said processed signal,
• comparing characteristics of each portion of said signal corresponding to a mechanical action with a reference model of the corresponding signal characteristics, the control unit comprising a memory in which is stored at least one reference model of said signal characteristics, said comparison comprising the estimation of a similarity index between the processed signal and the reference signal,
• determining, from said comparison, whether said at least one measuring of mechanical action and said at least one supplemental information actually belong to said predetermined code, and
• sending a predetermined order to said implanted device (3) if the applied pattern corresponds to the predetermined code,
said sensor (4) and said control unit (5) being implantable in said animal or human body.

2. The system according to claim 1, **characterized in that** said sensor(s) and said control unit are adapted for the detection of a predetermined posture taken by said animal or human body during a predetermined duration corresponding to said supplemental information.

3. The system according to one of claims 1 to 2, **characterized in that** said control unit comprises means for measuring and detecting the duration of each detectable supplemental information and of each mechanical action.

4. The system according to one of claims 1 to 3, **characterized in that** said control unit comprises means for further performing the steps of:
a) detecting said supplemental information in a portion of said signal that has a level below a predetermined threshold during a period having a duration comprised between a minimum and a maximum duration,
b) detecting said mechanical action in a portion of said signal that has a level above a predetermined threshold during a period having a duration comprised between a minimum and a maximum mechanical action duration after a supplemental information,
c) storing characteristics of said signal in a memory,
d) repeating steps (a) to (c) for each portion of said signal until the end of the pattern,
e) if said pattern does not correspond to said predetermined code, restarting to step (a) at any time until detecting a pattern corresponding to said predetermined code.

5. The system according to one of claims 1 to 4, **characterized in that** said predetermined order to send to the implanted device is selected from the group consisting of an activation or deactivation of said implanted device, an activation or deactivation of at least one function of said implanted device, a modification of at least one parameter of said implanted device, a modification of a therapy provided by said implanted device, a modification of the shape of said implanted device, and an activation or deactivation of a feature of at least one safety of said implanted device.

6. The system according to one of claims 1 to 5, **characterized in that** it comprises means for generating a feedback for the user when said predetermined order is sent to said implanted device.

7. The system according to one of claims 1 to 6, **characterized in that** said sensor(s) is (are) adapted for the measuring of a mechanical action selected from the group consisting of an external manual percussion on the body, over the implantation site of said sensor, a muscular contraction, vibrations, a pressure increase of a body cavity and a pressure increase on the skin over the implantation site of said sensor.

8. The system according to one of claims 1 to 7, **characterized in that** said controlled implanted device is an artificial sphincter and **in that** said predetermined order comprises opening or closing an occlusive element of said artificial sphincter.

9. The system according to one of claims 1 to 8, **characterized in that** it comprises means for periodically updating said reference model(s).

10. The system according to one of claims 1 to 9, **characterized in that** at least two different predetermined codes are stored into said memory, each of these codes, when applied to the body, leading to the sending of an associated specific order to the implanted device.

11. The system according to one of claims 1 to 10, further comprising a wireless communication system between the implantable device and an external wireless base adapted to configure the detection parameters of the predetermined codes.

12. The system according to one of claims 1 to 11, **characterized in that** said at least one sensor is selected from the group consisting of a 1-, 2- or 3-axis accelerometer, a gyroscope, a localization system, a pressure sensor and an electric commutator.

13. The system according to one of claims 1 to 12, wherein said sensor is not electrically supplied generating at least one electrical signal when a mechanical action is applied on said sensor.

14. An implantable device adapted to be implanted in an animal or human body, comprising a control system according to one of claims 1 to 13.

15. The implantable device according to claim 14, consisting of an artificial sphincter.
